# EUROPEAN PATENT SPECIFICATION

(11) **EP 1 297 743 B1**
(45) Date of publication and mention of the grant of the patent: **30.11.2005**
(21) Application number: 02078835.2
(22) Date of filing: 18.09.2002
(51) Int. Cl.: A01J 5/007, A01J 5/013

(54) **A device for separating milk from a dairy animal**
Vorrichtung zur Milchauswahl eines milchgebenden Tiers
Dispositif pour sélectionner le lait d'un animal laitier

(30) Priority: 28.09.2001 NL 1019060
(43) Date of publication of application: 02.04.2003
(73) Proprietor: Lely Enterprises AG, 6300 ZUG (CH)
(72) Inventor: Vijverberg, Helena Geralda Maria, 3141 GH Maassluis (NL); Espada Aventin, Elena, 2627 AD Delft (NL)
(74) Representative: Corten, Maurice Jean F.M.

(56) References cited:
- EP-A- 1 000 535
- WO-A-00/05943
- WO-A-97/47187
- WO-A-99/31965

## Description

The present invention relates to a device for separating milk from a dairy animal according to the preamble of claim 1.

Such a device is known from EP-A-1000535. The device known therefrom is provided with a measuring device in the form of a colour measuring system provided with one or more sensors comprising one or more sources irradiating the milk successively or simultaneously with radiation of one or more different wavelengths and/or different intensities, while, during at least a part of the time when the sources are in their switched-on position, one or more receivers establish the radiation intensity during a time interval. When the obtained measurement data indicate that the colour of the measured milk deviates from normal values, the relevant milk is separated. However, it has appeared that the known device sometimes draws a wrong conclusion on the basis of the colour measurements, so that e.g. suitable milk is not used for being processed further, but is discharged.

It is i.a. an object of the invention to provide a device for separating milk from a dairy animal by means of which the decision whether or not milk obtained is suitable for being processed further can be taken in an accurate manner.

According to the invention, for that purpose a device of the above-described type comprises the measures according to the characterizing part of claim 1. The invention is based on the insight that the measured value of the milk variable depends on the measured period, also called interval, even when the condition of the dairy animal remains unchanged. By including in the memory, according to the invention, various reference values for the milk variable, the reference values depending on the measured period, in other words being a function of the period i.e. the interval, it is possible to make a more accurate comparison of the measured values, so that a correct decision can be taken whether or not the milk is suitable for being processed further. Moreover, after comparison of the measured values with the reference values, it is possible to draw more correct conclusions in relation to the condition, respectively the health of the dairy animal. Determination of the period between two successive milking runs may take place by means of a clock measuring the period of time, or by means of a counter counting the number of cows having been milked between the two successive milking runs.

It is noticed that from WO 99/31965 it is known per se to vary a threshold value in dependence on the interval for determining the extent to which the udder of a dairy animal has been emptied.

The measuring device preferably comprises a colour sensor measuring system for measuring the intensity of at least one wavelength band, in particular in the visible wavelength range, of the milk obtained from the dairy animal, the variable being the intensity of the wavelength band. With the aid of the colour sensor measuring system especially the intensity of the separate colours in the milk obtained from the separate udder quarters is established. Therefore, in this embodiment the variable is constituted by the colour of the milk obtained.

In a further embodiment of a device according to the invention the measuring device is constituted by a conductivity meter known per se for measuring the conductivity of the milk obtained during the milking run. The conductivity meter preferably measures the conductivity of the milk obtained from the separate udder quarters.

In a still further embodiment of a device according to the invention the measuring device is constituted by a thermometer for measuring the temperature of the milk obtained during the milking run. The thermometer preferably measures the temperature of the milk obtained from the separate udder quarters.

In another further embodiment of a device according to the invention the measuring device is constituted by a component meter for measuring the quantity of a component of the milk obtained during the milking run, such as fat, protein, urea, bacteria, sugars, free fatty acids, germs, etc. The component meter preferably measures the components of the milk obtained from the separate udder quarters.

When the measuring device is suitable for measuring during the entire course of the milking run the value of the milk variable for obtaining a measurement pattern of the milk variable, and when the memory is suitable for storing the measurement pattern, it is possible to take a still more accurate decision whether or not the obtained milk is processed further. Comparing measurement patterns with reference patterns appears to result in more correct decisions than exclusively comparing one single measured value.

The processing device is in particular provided with an averaging device for determining the average of a measurement pattern of a milk variable, it being advantageous that the memory is suitable for storing the average measurement pattern. Such an average measurement pattern may excellently be used for determining deviations from this average pattern, which may be an indication that the condition of the dairy animal is different from normal or that the milk produced by the dairy animal is different from normal. Such an average measurement pattern appears to provide per animal a more accurate indication of the deviation than a predetermined reference value, especially when the average is a so-called progressive average, i.e. an average over e.g. the last ten milking runs (another number is possible as well).

In an embodiment of a device according to the invention the memory is suitable for storing a reference pattern.

Although for all the animals the same thresholds may be used, it is advantageous when the memory of the processing device contains an upper threshold pattern and/or a lower threshold pattern for a relevant measurement pattern of a milk variable for an animal.

In a further embodiment of a device according to the invention the processing device is provided with a comparing device for comparing a momentary measurement pattern of a milk variable with the stored measurement pattern of the milk variable, and for issuing a comparison signal indicative of the comparison result. It is thus possible, when the device is provided with a milk line system comprising a number of lines and with at least one device controlled by the comparison signal for guiding milk flowing through the milk line system to a relevant line, to discharge automatically unsuitable milk or to convey suitable milk for being processed further.

For the purpose of enabling visual checking it is advantageous when the device comprises a displaying device for displaying the comparison signal. When the device comprises a device for generating a warning, said warning device being controlled by the comparison signal, it is possible, in certain situations, to give a warning to the manager of the device, e.g. in the form of a sound signal.

The invention will be explained hereinafter in further detail with reference to an embodiment shown in the drawing, in which:
Figure 1 is a schematic view of a device for milking a cow, provided with a colour sensor measuring system, and
Figure 2 is a schematic view of a milking box with a milking robot provided with means for measuring a variable in relation to the cow.

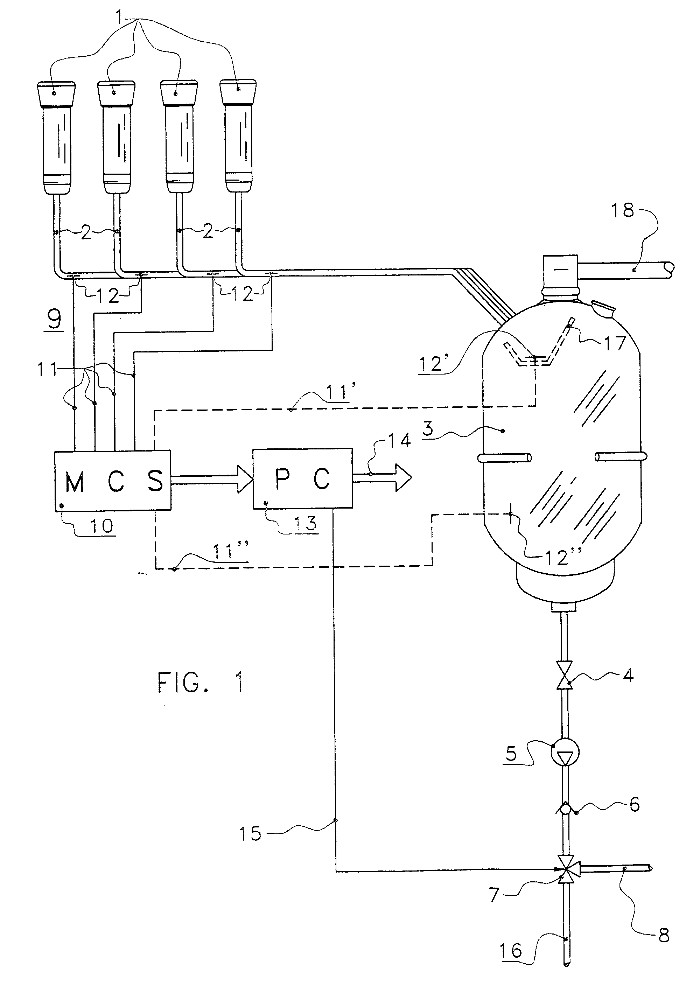

Figure 1 shows four teat cups 1 to be connected to the teats of an animal to be milked, the milk discharge lines 2 of said teat cups 1 debouching into a milk glass 3. To the milk glass 3 there is further connected a vacuum line 18 for the purpose of applying a vacuum in the milk glass 3 itself, in the milk discharge lines 2 and in the teat cups 1, said vacuum being required for keeping the teat cups connected to the teats of the animal, for enabling milking and for separating milk and air present therein from each other in the milk glass 3. From the milk glass 3 the milk obtained is discharged via a valve 4, a pump 5, a non-return valve 6 and a three-way valve 7 through a line 8 to a not further shown milk tank.

Figure 1 further shows a colour sensor measuring system 9, said measuring system comprising a colour intensity processing unit (MCS) 10, to which four sensors 12 are connected via glass fibre cables 11. Said sensors 12 are disposed in the milk lines 2 for establishing the intensity of a number of defined colours in the milk and for supplying signals representing these intensities to the processing unit 10. As a colour sensor measuring system may be used the Modular Color Sensor system CS1 of Stracon Messsysteme GmbH, Im Camisch 10, Kahla. The sensors used in this system are sensitive to frequencies in frequency bands for red (R), green (G) and blue (B). Therefore there are issued three signals per measurement, which may be considered as intensity values for these three colours.

Although until now the opinion prevailed that for milk of a constant composition these three intensity values have a fixed mutual relation, said relation depending i.a. on the impurities and components in the milk, it has appeared that the relation between the three intensity values depends on the interval, in other words depends on the period between two successive milking runs. Said period may be a period of time measured by a clock or a number of cows milked between two successive milking runs, said number being determined by a counter.

The colour intensity processing unit (MCS) 10 comprises a computer (PC) 13 (shown in the figure separately from the colour intensity processing unit (MCS) for the sake of clearness), in which for each animal to be milked there is a file in which all data required for milking a relevant animal are stored.

At each milking run also the obtained three intensity values of the relevant colours in the milk are stored. These intensity values stored at each milking run form the so-called historical intensity values. The progressive average may be determined from the historical intensity vales obtained for a certain animal during a defined number of the last milking runs carried out. Upon averaging milking runs with equal intervals should be used. The intensity values obtained at a next milking run with an equal interval may be compared with this progressive average, i.e. the last obtained intensity value of each of the three colours may be compared with the corresponding intensity value belonging to that interval, recorded in the computer as a progressive average. In other words, the intensity values are compared both mutually and with corresponding intensity values recorded during one or more previous milking runs with an equal interval. This comparison process takes place in the computer 13 which also functions as a comparing device. Subsequently the results of this comparison process may be displayed on a displaying device in such a manner that the presence of certain substances, such as impurities, in the milk can be read directly therefrom. These results may be supplied via the line 14 to a screen or to a printer.

Instead of determining the progressive average of the intensity values for each of the colours, it is also possible to determine in another manner for each colour a calibration value, such as in particular a reference pattern, respectively a lower threshold pattern or an upper threshold pattern. It is possible to apply calibration values which could hold for the milk obtained from all the animals or from a group of animals. In that case it will not be necessary to dispose a sensor 12 in each of the milk discharge lines 2, but an overflow reservoir 17 may be disposed in the milk glass 3, in which overflow reservoir there is provided such a sensor 12' which is connected to the processing unit 10 via a glass fibre cable shown by a "dashed" line 11'. As a further alternative a sensor 12" may be disposed in the lower part of the milk glass 3. Also in the latter case said sensor has to be connected to the processing unit 10 via a glass fibre cable 11".

However, in all situations it applies that, when inadmissible quantities of undesired substances appear to be present in the milk, the computer 13 issues a signal over the line 15 to the three-way valve 7, via which three-way valve 7 and the discharge line 16 connected thereto the milk containing these undesired substances may be discharged separately.

When for example blood has come into the milk, the intensity value issued by the sensor 12 for the colour red, will be higher than when no blood is present in the milk. This intensity value will then be higher than the progressive average established on the basis of the historical intensity values or higher than the calibration value applied (of course in dependence on the comparison with values belonging to the same interval). Also when there are no impurities in the milk, alterations in the concentration of substances normally being present in the milk may still be established. When for example the fat content of the milk changes in the course of the lactation period, then the mutual relation of the three intensity values established during each milking run changes as well.

Because the composition of the milk is different for different animals, which is even visually perceptible from the colour, the intensity values for the three colours will have a mutually different ratio for different animals. Therefore it is advantageous to determine the intensity values for each animal separately at each milking run and to compare them with calibration values or, in particular, with progressive averages established for this specific animal (and belonging to the same interval).

An example of the dependence of the measured colour intensity on the interval, said dependence having been proved clearly by means of the above-mentioned colour sensor measuring system, is given hereinafter. It has further appeared that this dependence is reproducible. For a particular cow it has appeared that the intensity of the blue frequency band rises in a particular manner when the period of time, the interval, increases or the number of cows having been milked increases. It has further appeared that the intensity of the green frequency band shows a certain, slight fall at an increasing interval. The intensity of the red frequency band showed a certain slight rise. For this cow the total sum of the intensities appeared to rise to a maximum value at an increasing interval and to fall via a particular pattern at a further increasing interval. The value of the intensity in the red frequency band reduced by the value of the blue frequency band appeared to show with this cow a falling pattern at an increasing interval, whereas the quotient of the intensity in the red frequency band and the intensity in the green frequency band rose to a maximum value at an increasing interval and remained constant at a further increase of the interval. It will be obvious that upon comparing the milk obtained from this cow, at each interval there has to be taken a different reference value or pattern to decide whether or not the milk obtained is suitable for being processed further.

It has further appeared that the colour intensity may differ per quarter, so that it is advantageous to compare the data per animal, per quarter, per interval, in order to be able to decide whether or not milk obtained from a quarter should be processed further.

It has further appeared that the flow of the milk obtained during the milking run depends on the interval. Also here, to be able to take a correct decision whether or not the milk obtained should be processed further, the measured flow values have to be compared with the reference value for that interval. It is noticed that a flow sensor for measuring the flow of the milk obtained during the milking run is known per se. In particular the flow sensor measures the flow of the milk obtained from the separate udder quarters. For the above-mentioned cow it has appeared that the flow rises at an increasing interval.

It has further appeared that the conductivity of the milk obtained for the mentioned cow rises at an increasing interval. A conductivity meter for measuring the conductivity of the milk obtained during the milking run, in particular per quarter, may then be used to take a correct decision whether or not the milk obtained (possibly per quarter) should be processed further.

It has further appeared that the temperature of the milk obtained for the mentioned cow rises at an increasing interval. In that situation a thermometer may be used for measuring the temperature of the milk obtained during the milking run, in particular for measuring the temperature of the milk obtained from the separate udder quarters, in order to take a correct decision whether or not the milk obtained (possibly per quarter) should be processed further.

Moreover it has appeared that for the mentioned cow the fat content of the milk obtained falls according to a certain curve at an increasing interval. Also for other components there appears to be a dependence between the quantity and the interval. A component meter for measuring the quantity of a component of the milk obtained during the milking run, such as fat, protein, urea, bacteria, sugars, free fatty acids, germs, etc., in particular the components of the milk obtained from the separate udder quarters, may then be used for taking a correct decision whether or not the milk obtained (possibly per quarter) should be processed further.

Besides it has appeared that for the mentioned cow the milk yield increases at an increasing interval. A quantity meter for measuring the quantity of the milk obtained during the milking run, in particular for measuring the quantity of the milk obtained from the separate udder quarters, may then be used in order to take a correct decision whether or not the milk obtained (possibly per quarter) should be processed further.

Research has also revealed that the activity of the mentioned cow, e.g. determined by means of a step counter, depends on the interval. Moreover it appears that an increased activity may indicate an udder inflammation, as a result of which the cow experiences pain and tries to kick off the teat cups during connection of the teat cups. Udder inflammation affects, as known, the quality of the milk, so that the activity may be used to determine the quality of the milk. The activity may also be determined in another manner, e.g. by measuring the heartbeat of the cow.

The above-mentioned relations have not only been found with a particular cow, but all cows appear to produce milk of which the measurable variables depend on the interval. Moreover cows also appear to show an interval-dependent activity. It will be obvious that the exact nature of that dependence can be determined by measurement.

As already mentioned above for colour intensity measurement, especially a measured measurement pattern (also called measured curve) of the variable appears to be adapted to decide during the milking run whether or not milk obtained should be processed further. This applies in particular to the pattern of colour, conductivity and flow during a milking run, although the other above-mentioned variables also show a pattern during the milking run, which pattern may be used for obtaining a correct decision whether or not milk obtained is suitable for being processed further.

In this situation an averaging device may determine the average of a measurement pattern of a milk variable and use this average as a reference pattern. Besides, other reference patterns are possible as well (e.g. an upper threshold pattern and/or a lower threshold pattern).

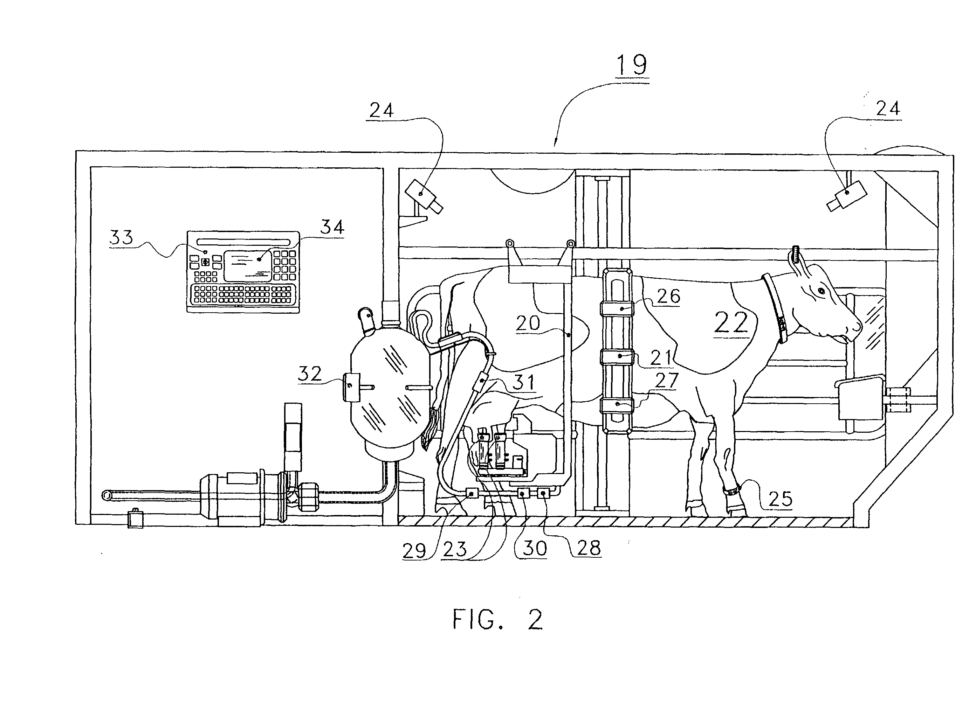

Figure 2 shows schematically a milking box 19 with a milking robot 20, to which the invention applies in particular. In this figure various measuring devices for measuring variables in relation to the cow are shown schematically.

For example the heart beat may be measured by means of a band 21 including a heart beat meter around the leg or the abdomen of the cow 22. Alternatively or additionally a heart beat meter known per se may be provided on the cow 22 near a place where an artery is located, in this connection the udder or an ear of the cow may be taken into consideration. A suitable heart monitoring system is for example obtainable at Polar Electro Oy, Helsinki, Finland. Alternatively a heart beat meter may be included in at least one of the teat cups 23.

In the milking box 19 there may be disposed one or more cameras 24 for observing and measuring the activity of the cow 22. The video pictures are analysed by movement recognition equipment known per se for determining activity parameters such as stepping, kicking and the like. To that end the picture is compared per cow 22 with stored historical data regarding the cow 22. Also in this situation it applies, as mentioned above, that the historical data used for the comparison relate to the same interval.

There may further be provided a step counter 25, a muscle contraction meter 26 and/or a muscle vibration meter 27 for determining the activity of the cow 22.

A flow sensor 28 measures the flow of the milk obtained during a milking run. A conductivity meter 29 measures the conductivity of the milk obtained during a milking run. A thermometer 30 measures the temperature of the milk obtained during a milking run. A component meter 31 measures the components, e.g. protein and fat, in the milk obtained during the milking run, and the milk yield is measured by a quantity meter 32 or yield meter.

All these measurement data are transmitted to or read by a processing device 33 comprising a computer having a memory. Besides the measurement data the processing device 33 also stores the period of time elapsed since the same animal has been milked. Alternatively the number of cows having been milked since the last milking run of the relevant cow is stored. To that end the processing device 33 comprises a clock (not explicitly shown, but implicitly present in the computer) for determining the period of time between two successive milking runs of the dairy animal. Alternatively the processing device comprises a counter for counting the number of cows. In the memory of the computer of the processing device 33 reference values or reference patterns are stored per interval, per animal or per group of animals, possibly per quarter, and per milk variable, respectively these reference values or reference patterns are generated by the system itself. The processing device 33 comprises a (non-shown) comparing device for comparing the measured value of the variable with the stored reference values. The comparing device issues a comparison signal, the value of which depends on the comparison result, and is thus indicative of the comparison result. This comparison signal may be displayed on a displaying device, such as a screen 34. As described above, the comparison signal may also be used for controlling a valve or the like, so that the milk obtained will be processed further or not. Should the comparison signal indicate a deviation, then it is also possible for the comparison signal to control a device for generating a warning (such as e.g. a loudspeaker) for issuing a signal (e.g. a sound) which is perceptible by a manager of the device.

It will be obvious that the measured values may be used separately, but that also combinations of measured values of different variables may be used for determining whether or not milk should be processed further (or for determining whether the condition of a dairy animal is within the standards). Thus a weight factor may be given to certain parameters for combining the measured values and/or comparison results obtained in a desired manner.

As described, Figure 2 shows a side view of a milking box 19 with a cow 22 present therein. The milking box 19 is provided with a milking robot 20 with teat cups 23 which are automatically connected to the teats of the cow 22 by means of the milking robot 20. Near the front side of the milking box 19 there is further disposed a feeding trough to which concentrate may be supplied in metered quantities. Other elements of the milking box and the robot are not shown in the figure for the sake of clearness.

The invention further relates to a method of separating milk from a dairy animal, said method comprising the step of determining the period (a period of time or a number of cows or another variable comprising a time aspect) between two successive milking runs of the dairy animal, the step of measuring a value of a variable in relation to the dairy animal and the step of issuing a signal indicative of the measured value, the step of storing a reference value in a memory, and the step of comparing the measured value of the variable with the reference value stored in the memory, and the step of separating milk in dependence on the result obtained during the comparison step, the method comprising the step of storing in the memory various reference values for the variable, the reference values depending on the measured period.

## Claims

1. A device for separating milk obtained from a dairy animal, said device being provided with a processing device (33) and with a measuring device (9, 29, 30, 31) for measuring a value of a milk variable and for issuing a signal indicative of the measured value to the processing device, the processing device (33) comprising a memory suitable for containing a reference value for the milk variable, and the processing device (33) comprising a comparing device for comparing the measured value of the milk variable with the reference values and for issuing a comparison signal, the device separating milk in dependence on the comparison signal, **characterized in that** the device is provided with a means for determining the period between two successive milking runs of the dairy animal, **in that** the memory contains various reference values for the milk variable, the reference values depending on the measured period, and **in that** the device is suitable for comparing the measured value with the corresponding reference value belonging to said determined period.

2. A device as claimed in claim 1, **characterized in that** the means for determining the period between two successive milking runs of the dairy animal is constituted by a clock for measuring the period of time between two successive milking runs.

3. A device as claimed in claim 1, **characterized in that** the means for determining the period between two successive milking runs of the dairy animal is constituted by a counter for counting the number of dairy animals having been milked since the last milking run of the relevant dairy animal.

4. A device as claimed in claim 1, 2 or 3, **characterized in that** the measuring device comprises a colour sensor measuring system (9) for measuring the intensity of at least one wavelength band, in particular in the visible wavelength range, of the milk obtained from the dairy animal, the variable being the intensity of the wavelength band.

5. A device as claimed in claim 4, **characterized in that** with the aid of the colour sensor measuring system the intensity of the separate colours in the milk obtained from the separate udder quarters is established.

6. A device as claimed in any one of the preceding claims, **characterized In that** the measuring device is constituted by a conductivity meter (29) for measuring the conductivity of the milk obtained during the milking run.

7. A device as claimed in claim 6, **characterized in that** the conductivity meter measures the conductivity of the milk obtained from the separate udder quarters.

8. A device as claimed in any one of the preceding claims, **characterized in that** the measuring device is constituted by a thermometer (30) for measuring the temperature of the milk obtained during the milking run.

9. A device as claimed in claim 8, **characterized in that** the thermometer (30) measures the temperature of the milk obtained from the separate udder quarters.

10. A device as claimed in any one of the preceding claims, **characterized In that** the measuring device is constituted by a component meter (31) for measuring the quantity of a component of the milk obtained during the milking run, such as fat, protein, urea, bacteria, sugars, free fatty acids, germs, etc.

11. A device as claimed in claim 10, **characterized in that** the component meter (31) measures the components of the milk obtained from the separate udder quarters.

12. A device as claimed in any one of the preceding claims, **characterized in that** the measuring device is suitable for measuring during the entire course of the milking run the value of the milk variable for obtaining a measurement pattern of the milk variable, and **in that** the memory is suitable for storing the measurement pattern.

13. A device as claimed in claim 12, **characterized in that** the processing device (33) is provided with an averaging device for determining the average of a measurement pattern of a milk variable.

14. A device as claimed in claim 13, **characterized in that** the memory is suitable for storing the average measurement pattern.

15. A device as claimed in claim 12, 13 or 14, **characterized In that** the memory is suitable for storing a reference pattern.

16. A device as claimed in any one of claims 12 through 15, **characterized In that** the memory of the processing device (33) contains an upper threshold pattern and/or a lower threshold pattern for a relevant measurement pattern of a milk variable for an animal.

17. A device as claimed in any one of claims 12 through 16, **characterized in that** the processing device (33) is provided with a comparing device for comparing a momentary measurement pattern of a milk variable with the stored measurement pattern of the milk variable, and for issuing a comparison signal indicative of the comparison result.

18. A device as claimed in claim 17, **characterized in that** the device is provided with a milk line system comprising a number of lines and at least one device controlled by the comparison signal for guiding milk flowing through the milk line system to a relevant line.

19. A device as claimed in claim 17 or 18, **characterized in that** the device comprises a displaying device (34) for displaying the comparison signal.

20. A device as claimed in claim 17, 18 or 19, **characterized in that** the device comprises a device for generating a warning, said warning device being controlled by the comparison signal.

## Patentansprüche

1. Vorrichtung zum Abscheiden der von einem milchgebenden Tier gewonnenen Milch, wobei die Vorrichtung ein Verarbeitungsgerät (33) und eine Meßvorrichtung (9, 29, 30, 31) aufweist, um einen Wert einer Melkvariablen zu messen und ein den gemessenen Wert anzeigendes Signal an das Verarbeitungsgerät zu geben, wobei das Verarbeitungsgerät (33) einen Speicher umfaßt, der einen Bezugswert für die Melkvariable enthält, und wobei das Verarbeitungsgerät (33) eine Vergleichsvorrichtung umfaßt, um den gemessenen Wert der Melkvariablen mit den Bezugswerten zu vergleichen und ein Vergleichssignal auszugeben, wobei die Vorrichtung in Abhängigkeit von dem Vergleichssignal Milch abscheidet,
**dadurch gekennzeichnet, daß** die Vorrichtung mit einer Vorrichtung zur Ermittlung des Abschnittes zwischen zwei aufeinanderfolgenden Melkdurchgängen des milchgebenden Tieres versehen ist, daß der Speicher verschiedene Bezugswerte für die Melkvariable enthält, wobei die Bezugswerte von dem gemessenen Abschnitt abhängen, und daß die Vorrichtung geeignet ist, den gemessenen Wert mit dem entsprechenden Bezugswert zu vergleichen, der zu dem ermittelten Abschnitt gehört.

2. Vorrichtung nach Anspruch 1,
**dadurch gekennzeichnet, daß** die Vorrichtung zur Ermittlung des Abschnittes zwischen zwei aufeinanderfolgenden Melkdurchgängen des milchgebenden Tieres durch eine Uhr gebildet ist, um den Zeitabschnitt zwischen zwei aufeinanderfolgenden Melkdurchgängen zu messen.

3. Vorrichtung nach Anspruch 1,
**dadurch gekennzeichnet, daß** die Vorrichtung zur Ermittlung des Abschnittes zwischen zwei aufeinanderfolgenden Melkdurchgängen des milchgebenden Tieres durch einen Zähler gebildet ist, um die Anzahl von milchgebenden Tieren zu zählen, die seit dem letzten Melkdurchgang des betreffenden milchgebenden Tieres gemolken worden sind.

4. Vorrichtung nach Anspruch 1, 2 oder 3,
**dadurch gekennzeichnet, daß** die Meßvorrichtung ein Farbsensormeßsystem (9) umfaßt, um die Intensität zumindest eines Wellenlängenbandes, insbesondere im sichtbaren Wellenlängenbereich, der von dem milchgebenden Tier gewonnenen Milch zu messen, wobei die Variable die Intensität des Wellenlängenbandes ist.

5. Vorrichtung nach Anspruch 4,
**dadurch gekennzeichnet, daß** mit Hilfe des Farbsensormeßsystems die Intensität der verschiedenen Farben in der von den verschiedenen Eutervierteln gewonnenen Milch ermittelt wird.

6. Vorrichtung nach einem der vorhergehenden Ansprüche,
**dadurch gekennzeichnet, daß** die Meßvorrichtung durch einen Leitfähigkeitsmesser (29) gebildet ist, um die Leitfähigkeit der während des Melkdurchganges gewonnenen Milch zu messen.

7. Vorrichtung nach Anspruch 6,
**dadurch gekennzeichnet, daß** der Leitfähigkeitsmesser die Leitfähigkeit der von den einzelnen Eutervierteln gewonnenen Milch mißt.

8. Vorrichtung nach einem der vorhergehenden Ansprüche,
**dadurch gekennzeichnet, daß** die Meßvorrichtung durch ein Thermometer (30) gebildet ist, um die Temperatur der während des Melkdurchganges gewonnenen Milch zu messen.

9. Vorrichtung nach Anspruch 8,
**dadurch gekennzeichnet, daß** das Thermometer (30) die Temperatur der von den einzelnen Eutervierteln gewonnenen Milch mißt.

10. Vorrichtung nach einem der vorhergehenden Ansprüche,
**dadurch gekennzeichnet, daß** die Meßvorrichtung durch ein Bestandteilsmeßgerät (31) gebildet ist, um die Menge eines Bestandteiles, wie z. B. Fett, Protein, Harnstoff, Bakterien, Zucker, freie Fettsäuren, Keime usw., der während des Melkdurchganges gewonnenen Milch zu messen.

11. Vorrichtung nach Anspruch 10,
**dadurch gekennzeichnet, daß** das Bestandteilsmeßgerät (31) die Bestandteile der von den einzelnen Eutervierteln gewonnenen Milch mißt.

12. Vorrichtung nach einem der vorhergehenden Ansprüche,
**dadurch gekennzeichnet, daß** die Meßvorrichtung geeignet ist, während der gesamten Dauer des Melkdurchganges den Wert der Melkvariablen zu messen, um ein Meßmuster der Melkvariablen zu erzielen, und daß der Speicher geeignet ist, das Meßmuster zu speichern.

13. Vorrichtung nach Anspruch 12,
**dadurch gekennzeichnet, daß** das Verarbeitungsgerät (33) mit einer Vorrichtung zur Ermittlung des Mittelwertes versehen ist, um den Mittelwert eines Meßmusters einer Melkvariablen zu ermitteln.

14. Vorrichtung nach Anspruch 13,
**dadurch gekennzeichnet, daß** der Speicher geeignet ist, das Durchschnittsmeßmuster zu speichern.

15. Vorrichtung nach Anspruch 12, 13 oder 14,
**dadurch gekennzeichnet, daß** der Speicher geeignet ist, ein Bezugsmuster zu speichern.

16. Vorrichtung nach einem der Ansprüche 12 bis 15,
**dadurch gekennzeichnet, daß** der Speicher des Verarbeitungsgerätes (33) ein oberes Grenzwertmuster und/oder ein unteres Grenzwertmuster für ein entsprechendes Meßmuster einer Melkvariablen für ein Tier enthält.

17. Vorrichtung nach einem der Ansprüche 12 bis 16,
**dadurch gekennzeichnet, daß** das Verarbeitungsgerät (33) mit einer Vergleichsvorrichtung versehen ist, um ein momentanes Meßmuster einer Melkvariablen mit dem gespeicherten Meßmuster der Melkvariablen zu vergleichen und ein das Vergleichsergebnis anzeigendes Vergleichssignal auszugeben.

18. Vorrichtung nach Anspruch 17,
**dadurch gekennzeichnet, daß** die Vorrichtung mit einem Milchleitungssystem versehen ist, das eine Anzahl von Leitungen und mindestens eine von dem Vergleichssignal gesteuerte Vorrichtung umfaßt, um durch das Milchleitungssystem strömende Milch in eine entsprechende Leitung zu leiten.

19. Vorrichtung nach Anspruch 17 oder 18,
**dadurch gekennzeichnet, daß** die Vorrichtung eine Anzeigevorrichtung (34) zum Anzeigen des Vergleichssignals umfaßt.

20. Vorrichtung nach Anspruch 17, 18 oder 19,
**dadurch gekennzeichnet, daß** die Vorrichtung eine Vorrichtung zur Erzeugung einer Warnung umfaßt, wobei die Warnvorrichtung durch das Vergleichssignal gesteuert wird.

## Revendications

1. Dispositif pour sélectionner le lait obtenu à partir d'un animal laitier, ledit dispositif étant pourvu d'un dispositif de traitement (33) et d'un dispositif de mesure (9, 29, 30, 31) pour mesurer une valeur d'une variable de lait et pour envoyer un signal indicatif de la valeur mesurée au dispositif de traitement, le dispositif de traitement (33) comprenant une mémoire appropriée pour contenir une valeur de référence pour la variable de lait, et le dispositif de traitement (33) comprenant un dispositif de comparaison pour comparer la valeur mesurée de la variable de lait aux valeurs de référence et pour envoyer un signal de comparaison, le dispositif séparant le lait suivant le signal de comparaison, **caractérisé en ce que** le dispositif est pourvu d'un moyen pour déterminer la période entre deux cycles de traite successifs de l'animal laitier, **en ce que** la mémoire contient diverses valeurs de référence pour la variable de lait, les valeurs de référence dépendant de la période mesurée, et **en ce que** le dispositif est approprié pour comparer la valeur mesurée à la valeur de référence correspondante appartenant à ladite période déterminée.

2. Dispositif selon la revendication 1, **caractérisé en ce que** le moyen pour déterminer la période entre deux cycles de traite successifs de l'animal laitier est constitué par une horloge pour mesurer la période entre deux cycles de traite successifs.

3. Dispositif selon la revendication 1, **caractérisé en ce que** le moyen pour déterminer la période entre deux cycles de traite successifs de l'animal laitier est constitué par un compteur pour compter le nombre d'animaux laitiers ayant été traits depuis le dernier cycle de traite de l'animal laitier concerné.

4. Dispositif selon la revendication 1, 2 ou 3, **caractérisé en ce que** le dispositif de mesure comprend un système de mesure capteur de couleur (9) pour mesurer l'intensité d'au moins une bande de longueur d'onde, en particulier dans la gamme de longueur d'onde visible, du lait obtenu à partir de l'animal laitier, la variable étant l'intensité de la bande de longueur d'onde.

5. Dispositif selon la revendication 4, **caractérisé en ce que**, à l'aide du système de mesure capteur de couleur, l'intensité des différentes couleurs dans le lait obtenu à partir des différents quartiers de pis est établie.

6. Dispositif selon l'une quelconque des revendications précédentes, **caractérisé en ce que** le dispositif de mesure est constitué par un appareil de mesure de conductivité (29) pour mesurer la conductivité du lait obtenu au cours du cycle de traite.

7. Dispositif selon la revendication 6, **caractérisé en ce que** l'appareil de mesure de conductivité mesure la conductivité du lait obtenu à partir des différents quartiers de pis.

8. Dispositif selon l'une quelconque des revendications précédentes, **caractérisé en ce que** le dispositif de mesure est constitué par un thermomètre (30) pour mesurer la température du lait obtenu au cours du cycle de traite.

9. Dispositif selon la revendication 8, **caractérisé en ce que** le thermomètre (30) mesure la température du lait obtenu à partir des différents quartiers de pis.

10. Dispositif selon l'une quelconque des revendications précédentes, **caractérisé en ce que** le dispositif de mesure est constitué par un appareil de mesure de composant (31) pour mesurer la quantité d'un composant du lait obtenu au cours du cycle de traite, tel que les matières grasses, les protéines, l'urée, les bactéries, les sucres, les acides gras libres, les germes, etc.

11. Dispositif selon la revendication 10, **caractérisé en ce que** l'appareil de mesure de composant (31) mesure les components du lait obtenu à partir des différents quartiers de pis.

12. Dispositif selon l'une quelconque des revendications précédentes, **caractérisé en ce que** le dispositif de mesure est approprié pour mesurer, au cours du cycle de traite entier, la valeur de la variable de lait pour obtenir une configuration de mesure de la variable de lait, et **en ce que** la mémoire est appropriée pour stocker la configuration de mesure.

13. Dispositif selon la revendication 12, **caractérisé en ce que** le dispositif de traitement (33) est pourvu d'un dispositif de détermination de moyenne pour déterminer la moyenne d'une configuration de mesure d'une variable de lait.

14. Dispositif selon la revendication 13, **caractérisé en ce que** la mémoire est appropriée pour stocker la configuration de mesure moyenne.

15. Dispositif selon la revendication 12, 13 ou 14, **caractérisé en ce que** la mémoire est appropriée pour stocker une configuration de référence.

16. Dispositif selon l'une quelconque des revendications 12 à 15, **caractérisé en ce que** la mémoire du dispositif de traitement (33) contient une configuration seuil supérieure et/ou une configuration seuil inférieure pour une configuration de mesure pertinente d'une variable de lait pour un animal.

17. Dispositif selon l'une quelconque des revendications 12 à 16, **caractérisé en ce que** le dispositif de traitement (33) est pourvu d'un dispositif de comparaison pour comparer une configuration de mesure momentanée d'une variable de lait à la configuration de mesure stockée de la variable de lait, et pour envoyer un signal de comparaison indicatif du résultat de comparaison.

18. Dispositif selon la revendication 17, **caractérisé en ce que** le dispositif est pourvu d'un système lactoduc comprenant un nombre de canalisations et au moins un dispositif contrôlé par le signal de comparaison pour guider le lait s'écoulant à travers le système de lactoduc jusqu'à une canalisation pertinente.

19. Dispositif selon la revendication 17 ou 18, **caractérisé en ce que** le dispositif comprend un dispositif d'affichage (34) pour afficher le signal de comparaison.

20. Dispositif selon la revendication 17, 18 ou 19, **caractérisé en ce que** le dispositif comprend un dispositif pour générer un avertissement, ledit dispositif d'avertissement étant contrôlé par le signal de comparaison.
